# EUROPEAN PATENT APPLICATION

(11) **EP 1 647 296 A1**
(43) Date of publication of application: **19.04.2006**
(21) Application number: 05256279.0
(22) Date of filing: 07.10.2005
(51) Int. Cl.: A61M 16/04, A61B 1/267

(54) **Airway tube guiding apparatus**

(30) Priority: 13.10.2004 JP 2004298424
(71) Applicant: MACHIDA ENDOSCOPE CO., LTD, Bunkyo-ku, Tokyo (JP)
(72) Inventor: Miyagi, Kunihiko, c/o Machida Endoscope Co, Ltd, Tokyo (JP)
(74) Representative: Hallybone, Huw George

(57) **Abstract**

A guiding apparatus to be used when inserting an airway tube from a mouth of a patient includes a body (A) and an endoscope (B) disposed through the body (A). The body (A) includes a blade (10). The blade (10) is inserted from the mouth of the patient to depress the epiglottis with a distal end portion thereof, exposing the glottis. The endoscope (B) includes an objective end portion (65), an ocular end portion (55) and an image guide (70) optically connecting the objective end portion (65) and the ocular end portion (55). The objective end portion (65) is disposed on the blade (10). The ocular end portion (55) is positioned out of alignment with the blade (10) either to the right or the left behind a rear end of the blade (10).

## Description

### BACKGROUND OF THE INVENTION

### Field of the Invention

This invention relates to a guiding apparatus to be used when inserting an airway tube into a patient's trachea.

### Description of Related Art

It is a known practice to insert an airway tube (endotracheal tube) into a trachea of a patient who had his or her airway obstructed in an emergency such as a traffic accident to open and maintain an airway, and to supply oxygen if needed. To help insert the tube, a guiding apparatus with a blade is commonly used. The blade is inserted into the mouth of the patient to depress the epiglottis with its distal end portion exposing the glottis. Then the tube is inserted from the mouth into the trachea, guided along the blade in the longitudinal direction.

Usually, an operator inserts the blade and the tube observing the throat, the epiglottis and the glottis of the patient while also illuminating them with illumination light from outside. The work can be difficult, requiring considerable operator skill.

Guiding apparatus as those disclosed in PCT International Publication WO2002/011608 and Japanese Patent Application Laid-Open No. 2002-564 have been invented to help ease the insertion. The guiding apparatus comprise a body having a blade and an endoscope disposed through the body. The endoscope has an objective end portion, an ocular end portion and an image guide connecting the objective and the ocular end portions. The objective end portion is disposed near a distal end portion of the blade and the ocular end portion is disposed behind the blade. An operator inserts the blade and the tube into a mouth of a patient, observing them through the ocular end portion. Insertion is easier than in a conventional way.

Such apparatus as those disclosed in the above-mentioned documents, however, tend to oblige the operator to assume an unnatural posture when inserting the blade and the tube while also observing them through the ocular end portion of the endoscope, because the ocular end portion of the endoscope is arranged behind and in alignment with the blade. Therefore, further improvements have been called for.

### SUMMARY OF THE INVENTION

The present invention has been accomplished in view of the above-mentioned problem. According to the present invention, there is provided a guiding apparatus to be used when inserting an airway tube through a mouth of a patient, comprising a body and an endoscope disposed on the body, the body including a blade which is to be inserted from the mouth of the patient to depress the epiglottis with its distal end portion exposing the glottis, the blade having a guiding surface, along which the tube is guided in the longitudinal direction, the endoscope having an objective end portion, an ocular end portion and an image guide optically connecting the objective end portion and the ocular end portion, the objective end portion being disposed on the blade and the ocular end portion being disposed behind the blade out of alignment with the blade either to the right or the left.

According to the features of the present invention, an operator can assume a natural posture when inserting the blade and the tube while observing them through the ocular end portion of the endoscope because the ocular end portion of the endoscope is disposed behind the blade out of alignment with the blade either to the right or the left, which makes a hand holding the apparatus and a head of the operator out of alignment with each other, thus improving workability.

Preferably, the body further includes a handle disposed parpendicular to the blade on a rear end portion of the blade and the ocular end portion is arranged out of alignment either to the right or the left with the plane on which the blade and the handle are disposed. This arrangement allows the operator to position the head and the hand holding the handle out of alignment with each other.

Preferably, the endoscope includes an ocular cylinder, one end portion of which being rotatably connected with the body by an attachment for rotation around a rotation axis orthogonal to the plane on which the blade and the handle are disposed, and the other end portion of which being provided as the ocular end portion. This arrangement enables the operator to move the ocular end portion to a desired angle position, making observation easier. This arrangement also allows the operator to maintain the ocular end portion at a desired angle position when turning the blade around its rear end portion to depress the epiglottis of the patient, which makes observation all the more easier.

Preferably, the attachment includes a hollow axial member, the axial member being fixed with either the ocular cylinder or the body and being rotatably supported by the other one, and the image guide is comprised of a bundle of optical fibers, the bundle of optical fibers extending through the ocular cylinder and the axial member of the attachment. This arrangement keeps the image guide from being exposed outside in the vicinity of the ocular cylinder, thus preventing it from disturbing insertion of the blade or the tube.

Preferably, the blade has a hollow receiving portion formed on it, the receiving portion extending from the rear end portion to an intermediate portion of the blade along the longitudinal direction thereof, the receiving portion being disposed adjacent to the guiding surface, the image guide extending through the receiving portion, the objective end portion of the endoscope being disposed at a window opened at a distal end surface of the receiving portion. This arrangement keeps the image guide from being exposed outside in the vicinity of the blade, thus preventing it from disturbing insertion of the blade or the tube.

### BRIEF DESCRIPTION OF DRAWINGS

FIG. 1 is a schematic side view of the guiding apparatus according to one embodiment of the present invention in an in-use condition.
FIG. 2 is a side view of the guiding apparatus of FIG. 1.
FIG. 3 is a cross-sectional view taken along line III-III of FIG. 2.
FIG. 4 is a view of the guiding apparatus viewed from the direction of arrow IV of FIG. 2.
FIG. 5 is an enlarged cross-sectional view taken along line V-V of FIG.
2.

FIG. 6 is an enlarged cross-sectional view of the area encircled as VI in FIG.2.

### DETAILED DESCRIPTION OF THE INVENTION

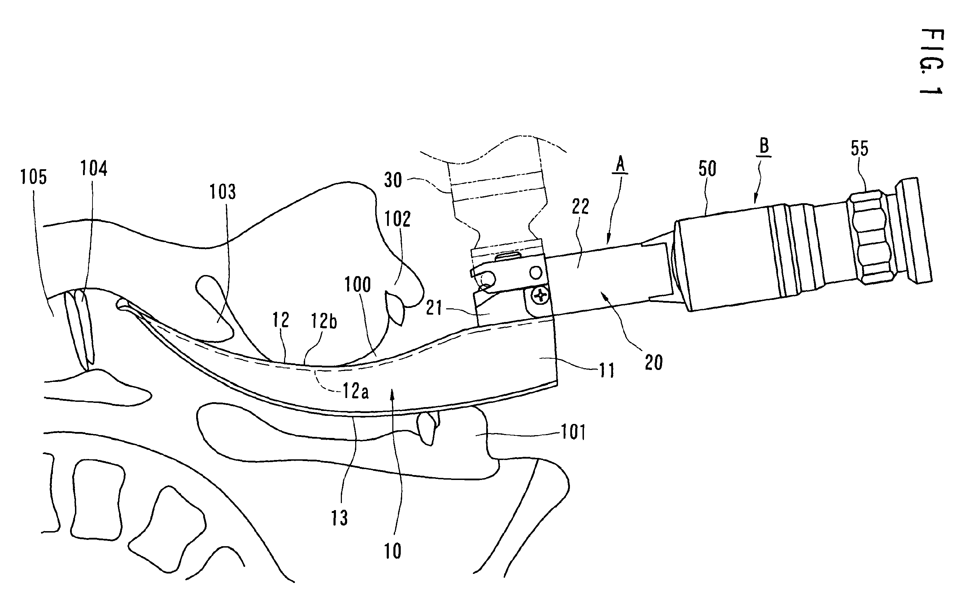

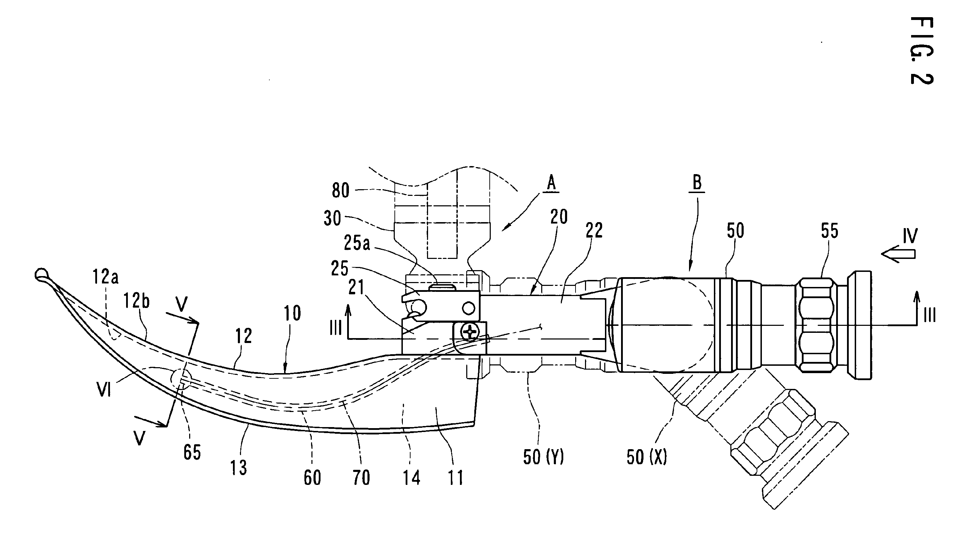

One preferred embodiment of an airway tube guiding apparatus according to the present invention will be described hereinafter with reference to the accompanying drawings. As shown in FIGS. 1 and 2, the guiding apparatus comprises a body A and an endoscope B. The body A includes a blade 10, a base 20 fixed on the rear end portion of the blade 10 and a handle 30 fixed on the base 20.

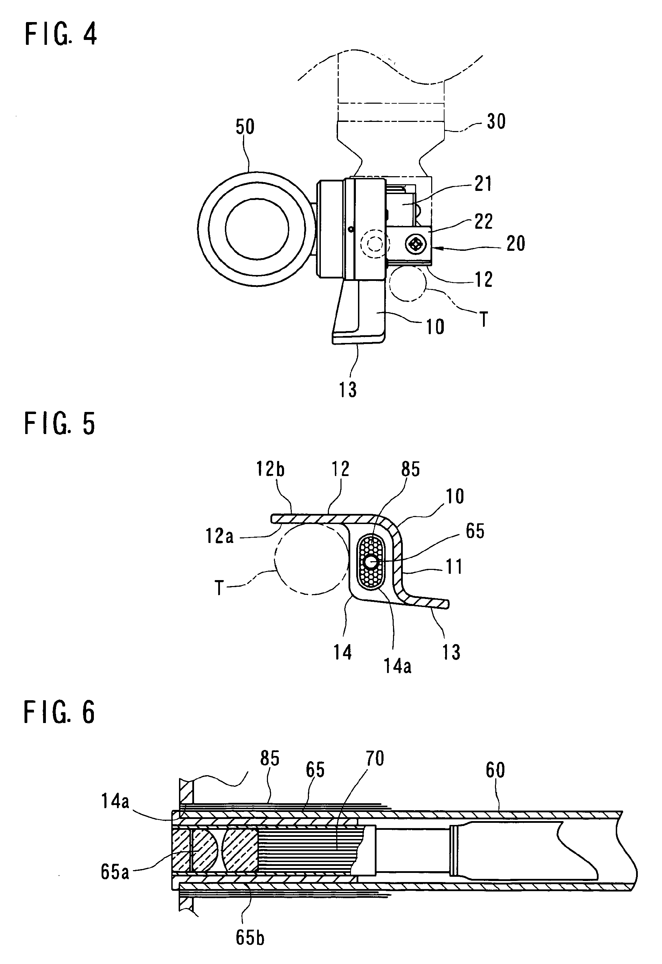

The blade 10 has a curved and tapered shape when viewed from a side as shown in FIGS. 1 and 2, and a generally Z-shaped cross section as shown in FIG. 5, and includes a flat plate-shaped main wall 11, a guiding wall 12 extending from one edge (an upper edge in an in-use condition) of the main wall 11 in a direction perpendicular to the main wall 11, and an abutment wall 13 extending from the other edge (a lower edge in an in-use condition) of the main wall 11 in a direction perpendicular to the main wall 11. The guiding wall 12 and the abutment wall 13 extend in opposite directions from each other. As shown in FIGS. 1 and 2, the guiding wall 12 and the abutment wall 13 are curved. One surface of the guiding wall 12 (a lower surface in an in-use condition) serves as a guiding surface 12a for a tube T to be described later, and the other surface of the guiding wall 12 (an upper surface in an in-use condition) serves as an abutment surface 12b.

The blade 10 includes a hollow receiving portion 14 formed integrally with the main wall 11 and the guiding wall 12. The receiving portion 14 is disposed adjacent to the guiding surface 12a and extends in a longitudinal direction of the blade 10, a distal end of which being located in an intermediate position of the blade 10, receded a predetermined distance from a distal end of the blade 10. The receiving portion 14 has generally the same height as the main wall 11, but its width is considerably small leaving enough width for the guiding surface 12a.

The base 20 includes a first member 21 and a second member 22. The first member 21 is fixed on an upper surface of a rear end portion of the guiding wall 12 of the blade 10. The second member 22, the front end portion of which being fixed on the first member 21, extends backward, in a direction opposite to the blade 10.

One end of the handle 30 is fixed on the first member 21. The handle 30 extends in a direction generally perpendicular to a direction in which the blade 10 and the second member 22 of the base 20 extend.

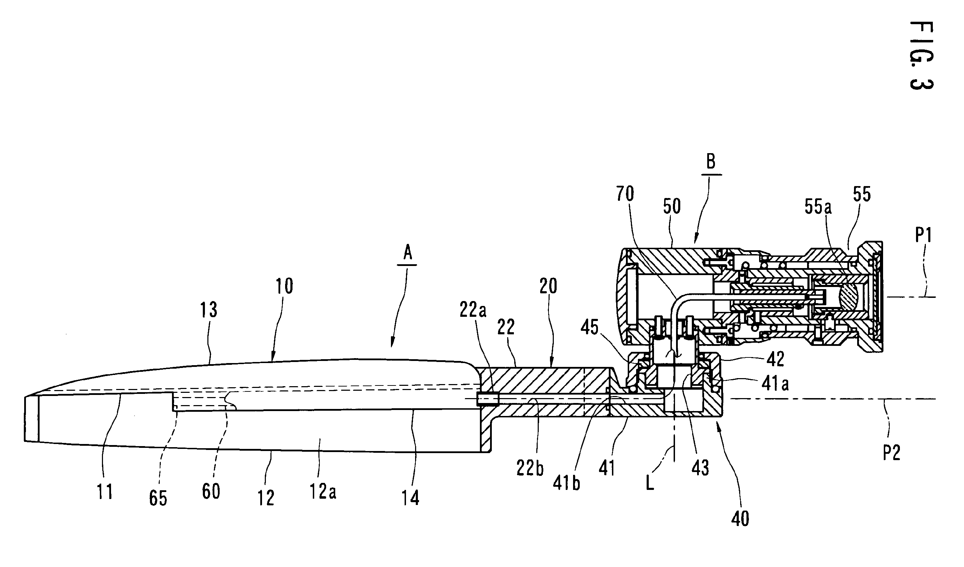

The endoscope B will be described hereinafter. As shown in FIG. 3, the endoscope B comprises an ocular cylinder 50 including an ocular end portion 55, an objective end portion 65 and an image guide 70 optically connecting the ocular end portion 55 and the objective end portion 65. The endoscope B further comprises an illumination light supply portion 80 for supplying illumination light (shown in FIG. 2 only) and an light guide 85 for transmitting illumination light (shown in FIG. 5 only). Detailed description of the components will be provided below.

As shown in FIG. 3, one end portion of the ocular cylinder 50 is connected rotatably around a rotation axis L to a rear end portion of the second member 22 of the base 20 by an attachment 40. A plane P1 in which a central axis of the ocular cylinder 50 lies is parallel to and distanced from a plane P2 in which the blade 10 and the handle 30 lie. The plane P1 is positioned to the left of the plane P2 when viewed from the operator in this embodiment. The rotation axis L is orthogonal to the planes P1 and P2.

As shown in FIG. 3, the attachment 40 includes a support member 41, a cover 42 and an axis member 43. The support member 41 is fixed on the rear end of the second member 22 of the base 20 and includes a short cylindrical portion 41a whose central axis coincides with the rotation axis L. The cover 42 is threadably mounted on the short cylindrical portion 41a. The axis member 43 is of a hollow cylindrical shape and its central axis coincides with the rotation axis L. The axis member 43 is fixed into a peripheral wall in the one end portion of the ocular cylinder 50 protruding from an outer circumferential surface of the ocular cylinder 50. A protruding end portion of the axis member 43 has a large diameter and it fits into the support member 41 and the cover 42, rotatably supported by them.

An annular rotation resistant member 45 intervenes between the protruding end portion of the axis member 43 and the cover 42, maintaining the ocular cylinder 50 at a desired angle position and making it non-rotatable unless a torque exceeding a predetermined value is applied. As shown in FIG. 2, the ocular cylinder 50 is restricted to rotate only up to 45 degrees clockwise (downwards) (to the position indicated with a reference numeral 50 (X)) and up to 180 degrees counterclockwise (to the position indicated with a reference numeral 50 (Y)) from a standard angle position (the position opposite to the blade 10) by rotation restriction pins (not shown) in this embodiment. When the ocular cylinder 50 is rotated 180 degrees counterclockwise and brought to the position indicated with the reference numeral 50(Y), it is overlapped with parts of the base 20 and the blade 10, reducing the dimensions of the guiding apparatus, thus making it easier to store and carry.

The other end portion of the ocular cylinder 50 is provided as the ocular end portion 55, in which an ocular lens system 55a is received. The ocular lens system 55a is opposed to a rear end surface of the image guide 70 comprised of a bundle of optical fibers.

As shown in FIGS. 2 and 3, the receiving portion 14 of the blade 10 accommodates a pipe 60 extending therethrough in a longitudinal direction. A rear end of the pipe 60 is inserted into and supported by a cylinder 22a fixed at a front end of the second member 22 of the base 20, while a front end of the pipe 60 is located at a center of an oval window 14a opened in a distal end wall of the receiving portion 14 as shown in FIGS. 5 and 6. A cylinder 65b accommodating an objective lens system 65a is inserted into and supported by a front end portion of the pipe 60. The objective end portion 65 of the endoscope B is comprised of the objective lens system 65a and the cylinder 65b.

A front end of the image guide 70 is accommodated in the cylinder 65b, with its tip reaching the objective lens system 65a. The image guide 70 extends through the pipe 60 to the rear and it further extends through a through-hole 22b formed in the second member 22 of the base 20, a through-hole 41b formed in the support member 41 of the attachment 40, the axis member 43, and the ocular cylinder 50, to the ocular lens system 55a.

As shown in FIG. 2, the illumination light supply portion 80 is accommodated in the handle 30. The illumination light supply portion 80 includes a lamp, a battery to supply power to the lamp and an optical system to collect illumination light from the lamp.

As shown in FIG. 2, a support 25 is fixed on the first member 21. The support 25 includes a short cylindrical portion 25a. A rear end portion of the light guide 85 comprised of a bundle of optical fibers is inserted into and supported by the short cylindrical portion 25a. The rear end surface of the light guide 85 is opposed to the optical system of the illumination light supply portion 80 to receive illumination light. The light guide 85 extends through the first member 21 of the base 20 and through the receiving portion 14, with its front end portion disposed at the window 14a surrounding the objective end portion 65 as shown in FIGS. 5 and 6.

The operation of the guiding apparatus as specified above will now be described. A patient is placed on his or her back. An operator holds the handle 30 of the guiding apparatus and inserts the blade 10 from a mouth 100 of the patient. More specifically, the blade 10 is inserted with the abutment wall 13 of the blade 10 abutted against the maxilla 101 and with the abutment surface 12b of the guiding wall 12 abutted against the submaxilla 102. Then the epiglottis 103 is depressed upward with the distal end portion of the blade 10 exposing the glottis 104.

Next, the airway tube T is inserted along the blade 10. The tube T is made of resin and of a curved shape. A distal end of the tube T proceeds forward, slidingly guided by the guiding surface 12a of the blade 10. The tube T can enter the trachea 105 through the glottis 104 because the epiglottis 103 is depressed by the blade 10 and the glottis 104 is exposed. After an airway has thus been established, the blade 10 is removed from the mouth 100 of the patient. Oxygen will be supplied through the tube T as needed.

During the insertion of the blade 10, the operator observes the pharynx, the epiglottis and the glottis of the patient through the ocular cylinder 50 of the endoscope B. The operator can see the distal end portion of the blade 10 because the objective lens system 65a of the endoscope B is disposed at the window 14a of the receiving portion 14 in the intermediate position of the blade 10. The operator holds the handle 30 with his or her right hand. The ocular cylinder 50's position out of alignment with the blade 10 and the handle 30 to the left improves workability because the head of the operator does not get in the way of the right hand of the operator holding the handle 30.

When the operator depresses the epiglottis 103 with the distal end portion of the blade 10, the blade 10 rotates slightly around its rear end portion. During the operation, the operator can keep the ocular cylinder 50 from rotating together with the blade 10 by holding it with the left hand because the ocular cylinder 50 is rotatable with respect to the body A. Therefore, the operator can operate the blade 10 while continuing observation without moving his or her head.

The head of the operator does not get in the way when the operator inserts the tube T while observing via the ocular cylinder 50 either, which also improves workability.

It should be noted that the present invention is not limited to the above embodiment but that many changes and modifications can be made without departing from the scope of the present invention. When the operator is left-handed, for example, the ocular cylinder 50 may be placed to the right of the plane P2 in which the blade 10 and the handle 30 are disposed.

## Claims

1. A guiding apparatus to be used when inserting an airway tube (T) from a mouth of a patient comprising a body (A) and an endoscope (B) disposed on said body:
said body (A) including a blade (10) to be inserted from the mouth of the patient to depress the epiglottis with distal end portion thereof, exposing the glottis, said blade having a guiding surface (12a), along which said tube is guided in the longitudinal direction; and
said endoscope having an objective end portion (65), an ocular end portion (55) and an image guide (70) optically connecting said objective end portion and said ocular end portion, said objective end portion (65) being disposed on said blade (10), said ocular end portion (55) being disposed behind said blade (10),
**CHARACTERIZED in that** said ocular end portion (55) is disposed out of alignment with said blade (10) either to the right or the left.

2. A guiding apparatus according to claim 1, wherein:
said body (A) further includes a handle (30) disposed perpendicular to said blade (10) at a rear end portion of said blade; and
said ocular end portion (55) is arranged out of alignment either to the right or the left with the plane (P2) in which said blade and said handle are disposed.

3. A guiding apparatus according to claim 2, wherein:
said endoscope includes an ocular cylinder (50), one end portion of said ocular cylinder being rotatably connected to said body (A) by an attachment (40) for rotation around a rotation axis (L) orthogonal to said plane (P2) in which said blade (10) and said handle (30) are disposed, and the other end portion of said ocular cylinder being provided as said ocular end portion (55).

4. A guiding apparatus according to claim 3, wherein:
said attachment (40) includes a hollow axial member (43), said axial member being fixed with either said ocular cylinder (50) or said body (A) and being rotatably supported by the other one; and
said image guide (70) is comprised of a bundle of optical fibers, said bundle of optical fibers extending through said ocular cylinder (50) and said axial member (43) of said attachment (40).

5. A guiding apparatus according to any one of the claims 1 to 4, wherein:
said blade (40) has a hollow receiving portion (14) formed thereon, said receiving portion extending from said rear end portion to an intermediate portion of said blade (10) along the longitudinal direction thereof, said receiving portion being disposed adjacent to said guiding surface (12a);
said image guide (70) extends through said receiving portion (14); and
said objective end portion (65) of said endoscope (B) is disposed at a window (14a) opened in a distal end surface of said receiving portion.
